# EUROPÄISCHE PATENTANMELDUNG

(11) **EP 3 001 918 A1**
(43) Veröffentlichungstag der Anmeldung: **06.04.2016**
(21) Anmeldenummer: 14187578.1
(22) Anmeldetag: 03.10.2014
(51) Int. Cl.: A24F 47/00, A61M 15/06, A61M 11/06

(54) **Inhalator**

(71) Anmelder: Sistro Präzisionsmechanik Ges.m.b.H., 6060 Hall in Tirol (AT)
(72) Erfinder: Winter, Eduard, 6060 Hall in Tirol (AT)
(74) Vertreter: Schwarz & Partner

(57) **Zusammenfassung**

Inhalator (1), umfassend ein Gehäuse (2), eine Verdampfungseinheit (8), ein Mundstück (4) und einen Taster (6), mit dem zwei Kontakte (10, 12) stromleitend verbindbar sind, wobei sich das Gehäuse (2) entlang einer Längsachse (L) erstreckt und der Taster (6) am einen Ende des Gehäuses (2) angeordnet ist, wobei der Taster (6) zum Verbinden der zwei Kontakte (10, 12) um die Längsachse (L) des Gehäuses (2) verkippbar ausgebildet ist.

## Beschreibung

Die vorliegende Erfindung betrifft einen Inhalator, umfassend ein Gehäuse, eine Verdampfungseinheit, ein Mundstück und einen Taster, mit dem zwei Kontakte stromleitend verbindbar sind. Weiters betrifft die Erfindung ein Bedienelement, umfassend ein Gehäuse und einen Taster, mit dem zwei Kontakte stromleitend verbindbar sind.

Gattungsgemäße Inhalatoren werden zur Verdampfung von Stoffen in der Verdampfungseinheit und anschließenden Inhalation über das Mundstück in unterschiedlichen Bereichen eingesetzt. Einsatzgebiete umfassen z.B. den medizinischen Bereich bei Lungen- oder Atemwegserkrankungen wie Asthma, Bronchitis, COPD oder dergleichen oder der Vorbeugung von Erkrankungen der Atemwege. Ein mögliches Einsatzgebiet betrifft auch sogenannte elektrische Zigaretten (E-Zigarette).

Der Taster verbindet zwei Kontakte stromleitend, um Stromfluss zur Verdampfungseinheit zu ermöglichen. Die Verdampfungseinheit weist eine Heizeinrichtung auf, die den zu verdampfenden Stoff erwärmt, welcher aufgrund des - in der Regel hohen Dampfdruckes - für die spätere Inhalation verdampft.

Beim Stand der Technik ist der Taster in der Regel an der Seite des Gehäuses angeordnet. Nachteilig daran ist, dass für die Bedienung des Inhalators das Gehäuse so in die Hand genommen werden muss, dass der Taster gedrückt und der Inhalator vom Benutzer bedient werden kann. Gegebenenfalls muss der Benutzer sogar beide Hände benutzen.

Aufgabe der vorliegenden Erfindung ist die Bereitstellung eines Inhalators, der aus praktisch jeder Position in einer Hand bedient werden kann.

Gelöst wird diese Aufgabe durch einen Inhalator, umfassend
- ein Gehäuse,
- eine Verdampfungseinheit,
- ein Mundstück und
- einen Taster, mit dem zwei Kontakte stromleitend verbindbar sind, welcher
dadurch gekennzeichnet ist, dass sich das Gehäuse entlang einer Längsachse erstreckt und der Taster am einen Ende des Gehäuses angeordnet ist, wobei der Taster zum Verbinden der zwei Kontakte um die Längsachse des Gehäuses verkippbar ausgebildet ist.

In einem Aspekt der Erfindung ist ein Bedienelement, umfassend
- ein Gehäuse und
- einen Taster, mit dem zwei Kontakte stromleitend verbindbar sind, vorgesehen, welches dadurch gekennzeichnet ist, dass sich das Gehäuse entlang einer Längsachse erstreckt und der Taster am einen Ende des Gehäuses angeordnet ist, wobei der Taster zum Verbinden der zwei Kontakte um die Längsachse des Gehäuses verkippbar ausgebildet ist.

Der Taster ist damit an einem stirnseitigen Ende des Gehäuses angebracht. Der Taster ist ein elektrischer Taster, wobei unter einem Taster ein Element verstanden wird, welches durch Drücken betätigbar ist und danach selbsttätig in die Ausgangslage zurückkehrt. Durch Drücken, d.h. Krafteinwirkung von Außen durch einen Benutzer wird der Taster betätigt und die beiden Kontakte werden in stromleitende Verbindung gebracht. Unter verkippbar gelagert wird im Sinne der Erfindung bevorzugt verstanden, dass das Gehäuse eine Längsachse L aufweist und der Taster eine Längsachse 1 aufweist, die entweder parallel zueinander angeordnet sind, oder - was bevorzugt ist - in Ruheposition zusammenfallen. Durch das Verkippen wird die Achse 1 des Tasters verdreht, sodass die Achsen 1, L dann einen von 0 verschiedenen Winkel zueinander einschließen.

Die nachfolgenden Ausgestaltungsvarianten gelten für Bedienelement und Inhalator gleichermaßen.

In einer bevorzugten Ausführungsvariante ist vorgesehen, dass das Gehäuse im Wesentlichen zylinderförmig ausgebildet ist. Dies ermöglicht eine einfach Handhabung und ein einfaches Festhalten von Bedienelement bzw. Inhalator.

Eine besonders bevorzugte Ausführungsvariante sieht vor, dass der Taster ein lösbar befestigbares Rastelement aufweist, welches ein Verkippen des Tasters um die Längsachse verhindert. Damit kann beim Transport verhindert werden, dass es zur Kontaktierung der Kontaktflächen kommt, sodass es in weiterer Folge insbesondere beim Inhalator zur Verdampfung bzw. Erwärmung kommt. Das Rastelement kann z.B. ein um die Längsachse drehbar gelagertes Element aufweisen. Das drehbar gelagerte Element kann derart ausgebildet sein, dass es durch Drehen eine Position einnimmt, die ein Verkippen des Tasters verhindert.

In einer Ausführungsvariante kann vorgesehen sein, dass der Taster abschraubbar ist und vorzugsweise einen Teil eines Batterie- oder Akkumulatordeckels bildet. Damit kann der Taster die Funktion eines Verschlusses eines Batteriefaches oder Akkumulatorfaches übernehmen. Demzufolge sieht eine Ausführungsvariante vor, dass das Gehäuse ein Fach für eine Batterie oder einen Akkumulator aufweist. Alternativ dazu kann die Stromversorgung natürlich über ein Anschlusskabel erfolgen, was aber aufgrund der Beschränkung des mobilen Einsatzes des Inhalators oder Bedienelements nicht bevorzugt ist.

Besonders bevorzugt ist der Taster derart ausgebildet, dass der Taster durch äußere Krafteinwirkung um die Längsachse des Gehäuses verkippbar ist und ohne Krafteinwirkung durch eine Federkraft in die Ausgangslage zurückgestellt wird. Dabei handelt es sich um einen mechanisch arbeitenden Taster, beim dem bei Betätigung Kontakte zueinander bewegt werden, um eine stromleitende Verbindung herzustellen. Im vorliegenden Fall werden die Kontakte beim Betätigen geschlossen, (also in Berührung gebracht), sodass man von einem Schließer spricht. Vom Taster zu unterscheiden ist der Schalter (oder Tasterschalter), der eine dauerhafte Zustandsänderung bewirkt, also einen dauerhaften Stromfluss - auch ohne äußere Krafteinwirkung - herstellt. Im vorliegenden Fall geht der Taster ohne äußere Krafteinwirkung in die Ursprungslage zurück und unterbricht damit automatisch wieder den Stromfluss.

In einer Ausführungsvariante ist vorgesehen, dass der Taster eine Schaltwippe, auf der ein Kontakt angeordnet ist, und eine Kontaktplatte aufweist. Schaltwippe und Kontaktplatte sind vorzugsweise symmetrisch um die Längsachse des Gehäuses. Die Schaltwippe weist bevorzugt ein Rückstellelement, beispielsweise ein federndes Druckstück auf.

Für den Inhalator gilt zusätzlich noch folgendes:

In einer bevorzugten Ausführungsvariante ist vorgesehen, dass das Mundstück am anderen Ende des Gehäuses - d.h. gegenüber dem Taster - angeordnet ist. Dies ermögliche eine gute Handhabung des Inhalators.

Bevorzugt handelt es sich beim Inhalator um eine E-Zigarette.

Die Verdampfungseinheit umfasst in der Regel ein Reservoir für den zu verdampfenden Stoff, der meist als Flüssigkeit vorliegt. Gegebenenfalls wird dieser Stoff mittels eines Dochts aufgesaugt. Eine Heizeinrichtung erhöht die Temperatur des Stoffs, sodass der Dampfdruck steigt und den Stoff dadurch verdampft.

Nachfolgend werden weitere Vorteile und Details der Erfindung anhand von Figuren erläutert.
- Fig. 1 zeigt: einen Schrägriss eines Inhalators.
- Fig. 2a, 2b, 2c zeigen: eine Seitenansicht (Fig. 2a), ein Ansicht auf den Taster (Fig. 2b) und einen teilweisen Schnitt durch das Gehäuse.
- Fig. 3a und 3b zeigen: den Taster im Längsschnitt in Ruheposition (Fig. 3a) und in betätigter Position (Fig. 3b).
- Fig. 4a und 4b zeigen: einen eine Explosionsdarstellung des Tasters aus zwei Ansichten.
- Fig. 5 zeigt: den Rastmechanismus für den Taster.

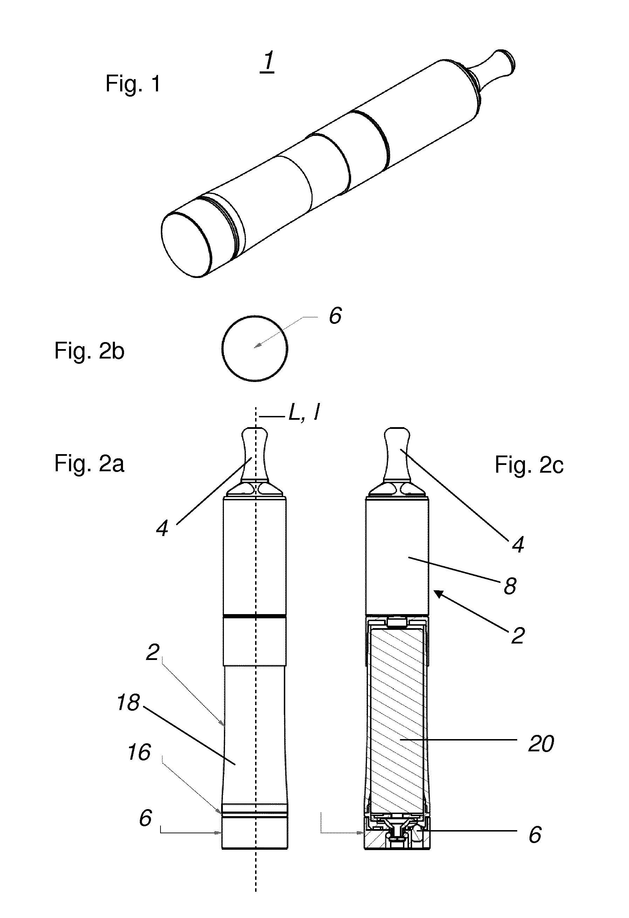

Fig. 1 zeigt in Schrägansicht einen Inhalator 1 bzw. auch ein Bedienelement gemäß der Erfindung. Die Fig. 2a bis 2c verdeutlichen den Inhalator 1 der Fig. 1. Der Inhalator 1 umfasst ein Gehäuse 2, das im Wesentlichen zylinderförmig ausgebildet ist. Das Gehäuse 2 erstreckt sich entlang der Längsachse L des Gehäuses. Weiters ist ein Mundstück 4 vorgesehen, über welches der Benutzer das Aerosol, das in einer Verdampfungseinheit 8 gebildet wird, inhalieren kann. Die Betätigung erfolgt über einen Taster 6, mit dem zwei Kontakte 10, 12 verbunden werden, sodass der Akkumulator 20 mit der nicht näher dargestellten Verdampfungseinheit 8 in Verbindung gebracht wird, womit diese Aerosol verdampfen kann.

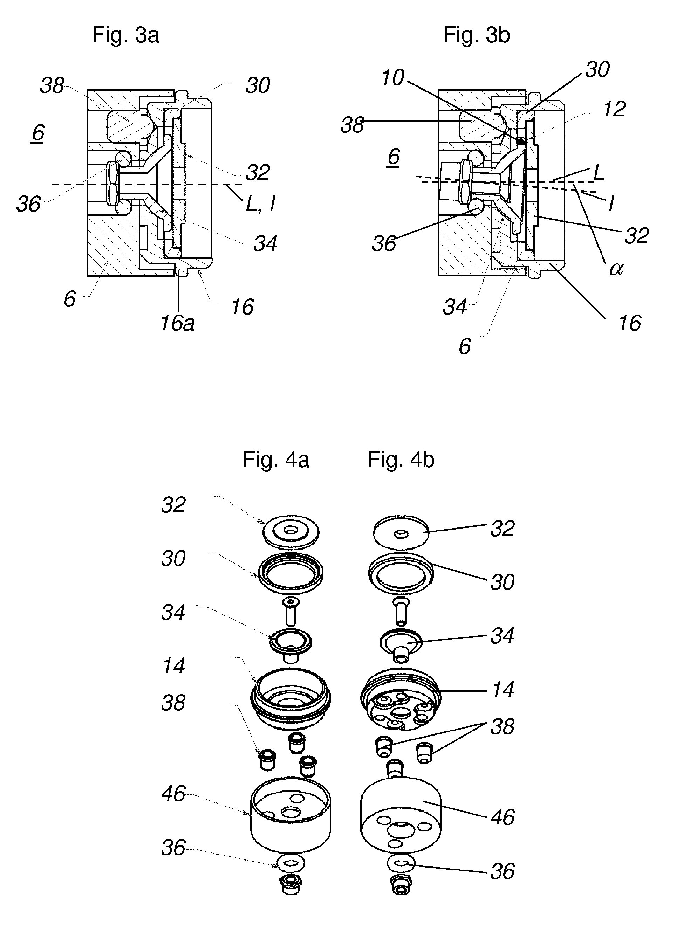

Der Taster 6 ist in Fig. 3a und Fig. 3b noch genauer erläutert. In Ruheposition (Fig. 3a) sind die beiden Kontakte 10, 12 voneinander getrennt. Ein Kontakt 12 sitzt auf der Schaltwippe 34, der andere Kontakt 10 auf der Kontaktplatte 32. Zur elektrischen Isolierung der beiden Kontakte 10, 12 ist eine Isolierscheibe 30 vorgesehen, die die Kontakte 10, 12 auf Distanz hält. Indem der Benutzer auf der Tastfläche 16a des Tasters 6 - insbesondere am Akkumulatordeckel 16 - drückt, wird der Taster 6 gegen die Längsachse L gekippt (siehe Fig. 3b) und es werden die Kontakte 10, 12 in Verbindung gebracht. Dabei wird das federnde Druckstück 38 durch die äußere Krafteinwirkung deformiert. Wird die äußere Kraft entfernt, bewegt das federnde Druckstück 38 den Taster 6 wieder in die Ausgangsposition zurück. Wie man aus der Darstellung gut erkennen kann, ist der Taster 6 derart ausgebildet, dass es keine Beschränkung der Kipprichtung gibt. Die Verkippung kann - betrachtet von der Längsachse L - in jedem Winkel von der Längsachse L weggekippt werden, was durch die Symmetrie von Schaltwippe 34 und Kontaktplatte 32 ermöglicht wird. Das Gehäuse 2 weist eine Längsachse L auf und der Taster 6 weist eine Längsachse 1 auf, die in Ruheposition (Fig. 3a) zusammenfallen. Durch das Verkippen wird die Achse 1 des Tasters 6 verdreht, sodass die Achsen 1, L dann einen von 0 verschiedenen Winkel α zueinander einschließen.

Der Taster 6 stellt also durch Krafteinwirkung mit den zwei Kontakten 10, 12 eine stromleitende Verbindung her. Sobald die Krafteinwirkung beendet wird, wird die Verbindung der beiden Kontakte 10, 12 wieder unterbrochen. Das Gehäuse 2 erstreckt sich entlang der Längsachse L und der Taster 6 sitzt am einen Ende des Gehäuses 2 stirnseitig angeordnet. Das Mundstück 4 sitzt am anderen Ende des Gehäuses 2, ebenfalls stirnseitig angeordnet.

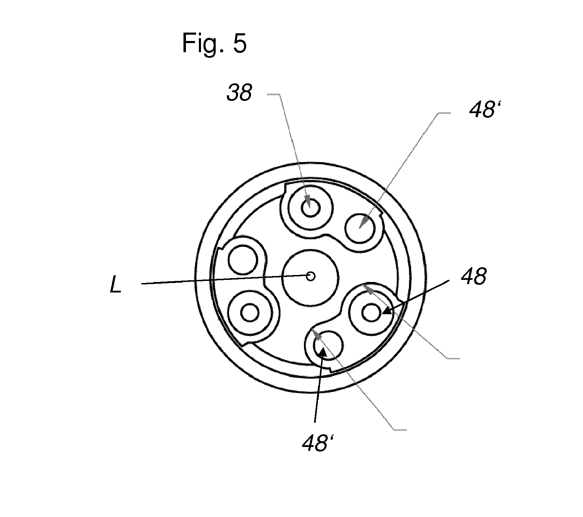

Der Taster 6 weist ein lösbar befestigbares Rastelement 14 aufweist, welches ein Verkippen des Tasters 6 um die Längsachse L verhindert, wenn das Rastelement 14 arretiert ist. In geöffneter Position erlaubt das Rastelement 14 die normale Betätigung des Tasters 6. Das Arretieren des Rastelements 14 kann ein unbeabsichtigtes Betätigen des Tasters 6 verhindern. Dies ist insbesondere beim Transport vorteilhaft. Erkennbar ist das Rastelement 14 in Fig. 4b und Fig. 5. Das Rastelement 14 kann verdreht werden, wodurch die federnden Druckstücke 38 teilweise verdeckt werden, was zur Arretierung führt, da sie durch die Abdeckung nicht mehr nachgeben können. Der Taster 6 ist in der Explosionsdarstellung der Fig. 4a und 4b aus zwei Perspektiven dargestellt. Erkennbart sind die Kontaktplatte 32, die den Kontakt 10 aufweist und die in eine Isolierscheibe 30 eingebettet ist. Darunter ist die Schaltwippe 34 erkennbar, auf der der zweite Kontakt 12 sitzt. Die Schaltwippe 34 ist in das Rastelement 14 eingebettet, welches wiederum in einem Tastergehäuse 46 sitzt. Das Rastelement 14 ist mit drei federnden Druckstücken 38 um die Längsachse verkippbar gelagert. Durch Deformation der Druckstücke 38 kann die Schaltwippe 34 aus der Ausgangsposition um die Längsachse L kippen. Die Druckstücke 38 nehmen ohne Krafteinwirkung wieder ihre normale Gestalt ein und kippen so die Schaltwippe 34 in die Ausgangsposition zurück.

Das Rastelement 14 weist eine Arretierung 50 mit Öffnungen 48, 48' unterschiedlicher Größe auf. Die Arretierung 50 ist um die Drehachse L rotierbar. Im nicht arretierten Zustand sind die größeren Öffnungen 48 genau oberhalb der federnden Druckstücke 38 angeordnet. Durch Verdrehen der Arretierung 50 werden die kleineren Öffnungen 48' über die federnden Druckstücke 38 angeordnet, was dem arretierten Zustand entspricht. Im nicht arretierten Zustand ragen die federnden Druckstücke 38 durch die größeren Öffnungen 48 durch und sind damit in Kontakt mit der Schaltwippe 34. Im nicht arretierten Zustand werden die federnden Druckstücke 38 von den kleineren Öffnungen 48' so abgedeckt, dass diese nicht mehr oder nicht mehr ausreichend mit der Schaltwippe 34 in Kontakt stehen.

Der Taster 6 ist abschraubbar und bildet einen Teil eines Batterie- oder Akkumulatordeckels 16.

## Patentansprüche

1. Inhalator (1), umfassend
- ein Gehäuse (2),
- eine Verdampfungseinheit (8),
- ein Mundstück (4) und
- einen Taster (6), mit dem zwei Kontakte (10, 12) stromleitend verbindbar sind,
**dadurch gekennzeichnet, dass** sich das Gehäuse (2) entlang einer Längsachse (L) erstreckt und der Taster (6) am einen Ende des Gehäuses (2) angeordnet ist, wobei der Taster (6) zum Verbinden der zwei Kontakte (10, 12) um die Längsachse (L) des Gehäuses (2) verkippbar ausgebildet ist.

2. Inhalator nach Anspruch 1, **dadurch gekennzeichnet, dass** das Gehäuse (2) im Wesentlichen zylinderförmig ausgebildet ist.

3. Inhalator nach Anspruch 1 oder Anspruch 2, **dadurch gekennzeichnet, dass** der Taster (6) ein lösbar befestigbares Rastelement (14) aufweist, welches ein Verkippen des Tasters (6) um die Längsachse (L) verhindert.

4. Inhalator nach einem der Ansprüche 1 bis 3, **dadurch gekennzeichnet, dass** der Taster (6) abschraubbar ist und vorzugsweise einen Teil eines Batterie- oder Akkumulatordeckels (16) bildet.

5. Inhalator nach einem der Ansprüche 1 bis 5, **dadurch gekennzeichnet, dass** das Gehäuse (2) ein Fach (18) für eine Batterie oder einen Akkumulator aufweist.

6. Inhalator nach einem der Ansprüche 1 bis 5, **dadurch gekennzeichnet, dass** das Mundstück (4) am anderen Ende des Gehäuses (2) angeordnet ist.

7. Bedienelement, umfassend
- ein Gehäuse (2) und
- einen Taster (6), mit dem zwei Kontakte (10, 12) stromleitend verbindbar sind,
**dadurch gekennzeichnet, dass** sich das Gehäuse (2) entlang einer Längsachse (L) erstreckt und der Taster (6) am einen Ende des Gehäuses (2) angeordnet ist, wobei der Taster (6) zum Verbinden der zwei Kontakte (10, 12) um die Längsachse (L) des Gehäuses (2) verkippbar ausgebildet ist.

8. Bedienelement nach Anspruch 7, **dadurch gekennzeichnet, dass** der Taster (6) durch äußere Krafteinwirkung um die Längsachse (L) des Gehäuses (2) verkippbar ist und ohne Krafteinwirkung durch eine Federkraft in die Ausgangslage zurückgestellt wird.

9. Bedienelement nach Anspruch 8, **dadurch gekennzeichnet, dass** das Gehäuse (2) im Wesentlichen zylinderförmig ausgebildet ist.
